Europäisches Patentamt

European Patent Office

Office européen des brevets

(11) Numéro de publication : **0 598 014 B1**

# (12) FASCICULE DE BREVET EUROPEEN

(45) Date de publication du fascicule du brevet :
20.09.95 Bulletin 95/38

(51) Int. Cl.⁶ : **G01N 33/544,** G01N 33/546, G01N 33/569, G01N 33/76

(21) Numéro de dépôt : **92917551.1**

(22) Date de dépôt : **30.07.92**

(86) Numéro de dépôt international :
**PCT/FR92/00749**

(87) Numéro de publication internationale :
**WO 93/03373 18.02.93 Gazette 93/05**

(54) **Utilisation d'atélocollagène comme support solide de fixation d'un capteur spécifique.**

(30) Priorité : **01.08.91 FR 9109819**

(43) Date de publication de la demande :
**25.05.94 Bulletin 94/21**

(45) Mention de la délivrance du brevet :
**20.09.95 Bulletin 95/38**

(84) Etats contractants désignés :
**AT BE CH DE DK ES FR GB GR IT LI LU MC NL SE**

(56) Documents cités :
**EP-A- 0 023 607
EP-A- 0 439 222
WO-A-83/03102
DE-A- 4 037 724
FR-A- 2 642 329
US-A- 4 169 804**

(73) Titulaire : **COLETICA
32, rue Saint Jean de Dieu
F-69007 Lyon (FR)**

(72) Inventeur : **FOURCART, Jean
Place de l'Orme
Baslieux Les Fimes
F-51170 Fimes (FR)**
Inventeur : **BUFFEVANT, Chantal
Les Carres
Millery
F-69390 Vernaison (FR)**
Inventeur : **HUC, Alain
26, chemin des Santons
F-69110 Ste-Foy-Les-Lyon (FR)**

(74) Mandataire : **Portal, Gérard et al
Cabinet Beau de Loménie
158, rue de l'Université
F-75340 Paris Cédex 07 (FR)**

## Description

La présente invention concerne l'utilisation d'atélocollagène comme support solide de fixation d'un capteur capable de réagir spécifiquement avec un élément à détecter dans un milieu biologique, un réactif et un procédé de mise en oeuvre.

Le document US YAPEL/3M US-A-4.169.804 décrit la fabrication de microparticules composites à réponse magnétique comprenant un matériau à réponse magnétique et une matrice insoluble dans l'eau, solide, poreuse et choisie parmi des matériaux protéiniques, des polysaccharides et leurs mélanges.

Le matériau à réponse magnétique est dispersé dans la matrice insoluble à l'eau, solide et perméable (voir l'abrégé et les revendications en colonnes 11 et 12).

Dans ce document, il est également prévu d'utiliser ces microparticules poreuses pleines comme matrice d'inclusion d'une enzyme insoluble dans l'eau (colonne 1, ligne 66 à colonne 2, ligne 2) ou encore d'un récepteur spécifique tel qu'une protéine ou un anticorps (voir colonne 2, ligne 63 à colonne 3, ligne 26).

Le déposant a déjà décrit dans le document FR-B1-2 642 329 l'utilisation de solutions d'atélocollagène et de glycosaminoglycanes pour la fabrication de microcapsules ainsi que des compositions cosmétiques, pharmaceutiques ou alimentaires en contenant.

Les présents inventeurs ont maintenant découvert que l'atélocollagène, et encore mieux un mélange d'atélocollagène et de polyholosides, constitue un support solide remarquable de fixation d'un capteur capable de réagir spécifiquement avec un élément à détecter dans un milieu biologique pour former un complexe spécifique qui peut alors être détecté par tout moyen de détection.

La présente invention a pour but principal de résoudre le nouveau problème technique consistant en la fourniture d'un support solide présentant sur sa surface extérieure un grand nombre de fonctions ou groupements réactifs, afin de permettre la fixation covalente d'un capteur spécifique, de préférence d'un capteur spécifique d'un élément à détecter dans un milieu biologique en particulier, dans des buts d'analyse et/ou de dosage qualitatif ou quantitatif.

La présente invention a encore pour but principal de résoudre le nouveau problème technique consistant en la fourniture d'un support solide de fixation à sa surface, d'un capteur spécifique qui présente de très bonnes propriétés mécaniques rendant possible une dispersion très aisée dans divers milieux, notamment grâce à une agitation vigoureuse sans détérioration sensible de ses capacités ou propriétés initiales.

La présente invention a pour but de résoudre le nouveau problème technique consistant en la fourniture d'un support solide de fixation d'un capteur spécifique d'un élément à détecter dans un milieu biologique qui soit capable de permettre un mélange rapide avec le milieu biologique ainsi qu'une séparation aisée de ce milieu biologique, afin d'aboutir à un gain de temps appréciable dans le cadre des analyses médicales en vue d'un diagnostic médical. L'invention résoud ces problèmes techniques pour la première fois, de manière simultanée, simple, aisée, fiable, peu coûteuse et utilisable à l'échelle industrielle et médicale.

L'invention permet avantageusement dans le cadre d'une application biologique, de réaliser des dosages sensibles et reproductibles de substance présente dans les fluides biologiques. L'invention trouve avantageusement également application dans le cadre de la détection d'un rotavirus humain ainsi que pour le titrage de l'HCG dans un fluide biologique tel que le sérum.

Ainsi, selon un premier aspect, la présente invention concerne l'utilisation d'atélocollagène, et encore mieux un mélange d'atélocollagène et de polyholoside comme support solide de fixation d'un capteur, dénommé ligand ou molécule d'affinité ("affinant"), capable de réagir spécifiquement avec un élément à détecter dans un milieu biologique pour former un complexe spécifique pouvant ensuite être détecté par tout moyen de détection.

Selon une caractéristique avantageuse de cette utilisation, l'atélocollagène, ou le mélange atélocollagène-glycosaminoglycane est utilisé sous forme de particules, billes, ou capsules, de préférence de diamètre compris entre environ 5 et 500 μm (micromètres). On préfère des capsules et en particulier des microcapsules.

Selon une caractéristique avantageuse de l'invention, l'atélocollagène est combiné à un polyholoside, avantageusement la proportion relative de polyholoside par rapport à l'atélocollagène varie entre 15 et 50 % en poids. Le polyholoside peut être par exemple un glycosaminoglycane, du dextrane ou du chitosane.

Ce support solide lorsqu'il s'agit d'atélocollagène, peut être fabriqué sous forme de particules par coagulation de gouttes.

Lorsqu'il s'agit d'un mélange d'atélocollagène et d'un polyholoside, les particules solides sont de préférence obtenues par réticulation interfaciale comme décrit dans le brevet précédent du déposant FR-B1-2 643 329.

Selon une autre caractéristique avantageuse de l'invention, l'atélocollagène ou le mélange atélocollagène-polyholoside sous forme de particules, billes ou capsules contient un oxyde magnétique, de préférence un oxyde magnétique de fer.

Avantageusement, la concentration d'oxyde magnétique est comprise entre 0,1 et 10% en poids par rapport au poids total des particules.

Selon une autre caractéristique particulière de l'invention, le capteur précité est fixé de manière covalente par un agent chimique sur les particules de collagène formant support solide de fixation.

Le support solide selon l'invention présente l'avantage de présenter des fonctions chimiques de surface telles que les fonction aminées ($-NH_2$), carboxyliques ($-CO_2H$) ou alcooliques ($-OH$), qui permettent de réaliser le couplage du capteur formant ligand ou molécule d'affinité ("affinant").

Dans le cas des fonctions aminées ($-NH_2$), on utilisera de préférence comme agent chimique de couplage entre le capteur et le support solide, un réactif de type NHS (N-hydroxysuccinimide) qui peut réagir avec le groupement aminé du support solide et avec le capteur formant ligand ou affinant. Ce couplage est effectué de préférence en deux étapes, la première étape consistant à fixer le réactif à la surface des particules solides du support, puis, après avoir éliminé l'excès de réactif chimique de couplage, à réaliser le couplage du capteur formant ligand ou affinant avec les particules de support solide obtenues après la première étape ci-dessus.

Les agents chimiques de couplage sont dits homobifonctionnels si le deuxième groupement réactif est également de type NHS ou hétérobifonctionnels si ce dernier est différent. Un exemple de deuxième groupement réactif différent est un groupement maléimide pouvant fixer une molécule par une fonction thiol ($-SH$).

Dans le cas des fonctions carboxyliques, les réactifs chimiques de couplage utilisent de préférence des agents du type carbodiimide hydrosolubles. Leur utilisation se fait également par un procédé en deux étapes. Les carbodiimides sont des agents hétérobifonctionnels qui donnent une liaison entre une fonction carboxylique ($-COOH$) et une fonction aminée ($-NH_2$).

Dans le cas des fonctions alcooliques, la technique utilisée, de préférence, comprend une oxydation de la fonction alcool en un aldéhyde (CHO) par un agent d'oxydation douce, tel que le périodate de sodium ($NaIO_4$). L'aldéhyde peut ensuite réagir avec une fonction aminée ($-NH_2$) en formant une base de Schiff. Ainsi, ce couplage se fait encore par un procédé en deux étapes, la première étape consistant à réaliser l'oxydation de la fonction alcool en aldéhyde, la deuxième étape consistant à réaliser le couplage proprement dit de l'agent formant ligand ou affinant avec les particules solides du support obtenu à la fin de la première étape.

Il est à noter que grâce à l'emploi comme support solide d'atélocollagène ou de mélange atélocollagène-polyholoside, on obtient la présence de diverses fonctions de surface sur les particules du support solide, en quantité importante, ce qui rend aisé un couplage de populations moléculaires différentes sur la même surface, par exemple un anticorps et une enzyme.

Pour ce faire, on pourra utiliser deux méthodes, à savoir :

- le mélange de populations moléculaires différentes lors de la phase de couplage ce qui donnera lieu à la fixation à la surface des particules du support solide d'une partie variable de ces populations en fonction de la réactivité propre de chaque population et de leur concentration respective.

Le résultat sera alors une fixation en une quantité déterminée de chaque population et ceci de manière reproductible si les conditions de réaction sont reproduites.

D'autre part, l'activation successive des différentes fonctions disponibles sur la surface des particules solides, suivie du couplage séquencé des différentes populations moléculaires, permet alors d'obtenir un support solide à spécificité multiple.

D'autre part, l'activation successive des différentes fonctions disponibles sur la surface des particules solides, suivie du couplage séquencé des différentes populations moléculaires, permet alors d'obtenir un support solide à spécificité multiple.

On observera par ailleurs que, dans le cadre de l'invention, le support solide est fabriqué à partir de substances naturelles extraites de tissus animaux. Ceci est le cas de l'atélocollagène, qui est une protéine à longue chaîne extraite du tissu dermique, ainsi que du polyholoside, en particulier un glycosaminoglycane.

Grâce à l'utilisation selon l'invention d'atélocollagène et encore mieux d'un mélange d'atélocollagène-polyholoside, par fixation d'un capteur formant liant ou affinant, on obtient ainsi un réactif de détection qualitative et de mesure quantitative d'un élément quelconque d'un milieu d'origine biologique. La notion d'élément recouvre ici dans le cas de la présente description et des revendications toute espèce d'anticorps ou d'antigènes, sous forme soluble ou particulaire, ainsi que toutes les espèces moléculaires pouvant donner lieu à des complexes spécifiques pour lesquels la constante d'affinité est suffisamment forte pour déplacer la réaction dans le sens de la formation du complexe.

Ce réactif peut être utilisé de manière classique dans le cadre des procédés de détection qualitative et de mesure quantitative d'élément d'un milieu d'origine biologique, dans le cadre des analyses médicales en vue d'un diagnostic médical. La manipulation est aisée et aboutit à un gain de temps appréciable grâce à un mélange rapide avec le milieu biologique et une séparation aisée des particules solides de ce milieu biologique.

Ce réactif permet de mettre en oeuvre des procédés classiquement dénommés "immuno-essais" en phase hétérogène.

Le réactif selon l'invention permet de détecter des éléments présents en quantité infinitésimale dans un milieu complexe d'origine biologique grâce à la capacité d'extraction et de séparation de l'élément recherché par le capteur formant ligand ou affinant fixé sur la phase solide.

Le milieu d'origine biologique est habituellement une phase liquide constituée par un liquide d'origine biologique tel que le sang, l'urine, le liquide céphalo-rachidien, etc. ; ou un extrait de tissu nécessitant la mise en oeuvre d'un moyen d'extraction de nature physique tel que la chaleur, un rayonnement, etc. ; ou chimique tel qu'une enzyme, un détergent, un acide, une base, etc.

Le capteur formant agent, liant ou affinant fixé sur la phase solide ou support solide selon l'invention, est spécifique de l'élément recherché et de tels capteurs sont bien connus de l'homme de l'art. Ces capteurs peuvent donner lieu dans des conditions physico-chimiques adéquates à la formation de complexes stables notamment par des réactions de type immunitaire, qui sont fixés à la surface de la phase solide, complexes que l'on peut ainsi isoler des autres populations moléculaires en présence par un procédé quelconque aboutissant à la séparation de la phase solide comportant le complexe lié à celle-ci.

La révélation de la présence du complexe peut être ensuite effectuée par mesure de la consommation, par exemple par une technique de compétition, ou de la quantité proportionnellement fixé d'un traceur marqué qui donnera un signal spécifique mesurable à l'aide d'un procédé chimique ou physique tel que le comptage isotopique, la spectrophotométrie, la luminométrie, l'ampérométrie, etc. Toutes ces technique sont bien connues à l'homme de l'art.

Selon un deuxième aspect, l'invention couvre ainsi à titre de produits nouveaux, un réactif biologique caractérisé en ce qu'il comprend comme support ou phase solide, des particules d'atélocollagène, et encore mieux un mélange d'atélocollagène et de polyholoside.

Diverses variantes de réalisation de ce réactif résultent de la description précédente faite en relation avec le premier aspect de l'invention, et résulteront également de la description qui va suivre en référence à divers exemples non limitatifs de l'invention.

Selon un troisième aspect, la présente invention couvre encore un procédé de détection dans un milieu biologique d'un élément à détecter.

Selon un mode de réalisation avantageux, l'invention concerne un procédé pour la détection d'un rotavirus humain, caractérisé en ce qu'on utilise un réactif biologique dont le capteur comprend un anticorps anti-rotavirus humain.

Selon un autre mode de réalisation particulier, l'invention concerne un procédé pour le titrage de l'HCG dans le sérum, caractérisé en ce qu'on utilise un réactif biologique dont le capteur comprend un anticorps anti-HCG.

Des variantes de réalisation de ce procédé sont également apparentes de la description précédente, ainsi que de la description suivante faite en référence aux exemples ci-après donnés simplement à titre d'illustration.

Comme polyholosides, on utilise en particulier des glycosaminoglycanes qui sont avantageusement des glycosaminoglycanes de structure en particulier choisis parmi le groupe constitué par le chondroïtine-4-sulfate, le chondroïtine-6-sulfate, le dermatane-sulfate, l'héparane-sulfate et le kératane-sulfate ainsi que l'héparine et ses dérivés, en particulier les dérivés de bas poids moléculaire dont le poids moléculaire peut varier entre 2 000 et 10 000. Comme polyholosides, on peut également citer le chitosane et le dextrane.

Le polyholoside est en particulier utilisé sous forme de solution aqueuse à 0,5 à 4%, de préférence 0,5 à 2% ou encore de préférence environ 1%, en poids, par rapport à la solution de polyholoside.

L'atélocollagène est de préférence utilisé en solution aqueuse ayant une concentration en atélocollagène comprise entre 0,5 et 2 % en poids. Cette solution d'atélocollagène peut être obtenue en dissolvant des fibres d'atélocollagène dans une solution aqueuse légèrement acide. Cette solution aqueuse légèrement acide peut être de préférence une solution d'acide aqueuse d'acide acétique 0,1 M. L'atélocollagène peut être obtenu par la technique de digestion enzymatique de collagène bien connue de l'homme de l'art.

Pour la préparation du mélange atélocollagène-polyholoside, la solution de polyholoside est avantageusement introduite dans la solution d'atélocollagène. Ensuite, pour la préparation de particules solides, en particulier des microcapsules, on utilise un procédé de fabrication par réticulation interfaciale tel que décrit dans le document FR-B1-2 642 329.

Selon ce procédé, après avoir mélangé la solution d'atélocollagène, avec la solution de polyholoside, de manière homogène, on disperse une phase huileuse contenant une agent de réticulation sous forme de phase dispersée dans la phase continue formée par la solution d'un mélange d'atélocollagène, et de polyholoside, sous agitation pendant le temps nécessaire pour réaliser un degré approprié de réticulation interfaciale, ce qui permet de produire des microcapsules dont la paroi est une paroi mixte d'atélocollagène, et de polyholoside réticulé.

Les microcapsules sont séparées de manière appropriée par une décantation naturelle après un ou plusieurs lavages éventuels.

On peut également procéder par une extrusion laminaire au travers d'une buse d'extrusion, l'écoulement laminaire étant soumis à des vibrations de manière à former des gouttelettes individuelles qui tombent ensuite dans un bain de réticulation contenant l'agent réticulant.

Comme agent de réticulation, on utilise avantageusement un chlorure d'acide, un anhydride d'acide, ou un acide carboxylique dibasique ou polybasique. Un agent de réticulation préféré est choisi parmi le groupe constitué par le chlorure de téréphtaloyle, le chlorure de phtaloyle, le chlorure de sébacoyle, le chlorure de succinyle, le chlorure d'un acide tricarboxylique tel que l'acide citrique ou un anhydride d'acide tel que l'anhydride succinique.

Comme solvant de l'agent de réticulation, on utilise un liquide hydrophobe dans lequel l'atélocollagène et/ou le polyholoside, sont insolubles. On utilise encore de préférence du cyclohexane ou du chloroforme.

Grâce à l'emploi d'un procédé de réticulation interfaciale, on peut ainsi préparer des microcapsules qui peuvent encapsuler un principe actif quelconque. En particulier, il est avantageux d'encapsuler dans les microcapsules une particule magnétique composée par exemple d'un oxyde de fer, ce qui permet de faciliter la séparation du support solide des solutions dont on veut réaliser l'analyse, ce qui constitue un avantage technique important de l'invention. En effet, on obtient un temps de séparation inférieur à 5 s sur 1 cm de distance en présence d'un simple aimant permanent. Ceci est dû à leur masse relativement importante liée à une densité voisine de 1 ne nécessitant qu'une faible charge en oxyde magnétique de l'ordre de 1% en poids, pour obtenir ce résultat. L'agitation magnétique devient alors possible. Le support solide selon l'invention présente un volume relativement important ce qui lui apporte une insensibilité aux forces d'agrégation, qu'elles soient hydrophobes ou ioniques. Cela permet encore la séparation rapide en cours de fabrication par centrifugation à basse vitesse, par exemple de l'ordre de 800 g pendant 2 min, et une remise en suspension sous forme non agrégée par simple agitation.

Les particules de support solide selon l'invention peuvent être obtenues avec une taille intermédiaire par exemple de l'ordre de 50 $\mu$m, grâce au procédé de fabrication utilisé, en particulier la réticulation interfaciale, ce qui permet d'obtenir des surfaces réactives suffisantes lors d'un immuno-essai (par exemple 625 mm$^2$ pour 50 $\mu$l de particules de support solide en suspension à 10% en poids). Cette surface extérieure est en permanence en contact avec le liquide réactionnel. C'est donc une surface extrêmement efficace.

Les particules du support solide selon l'invention sont par nature hydrophiles en raison de leur constitution chimique. On sait que les polyholosides sont des enchaînements de sucres possédant dans leur structure de nombreuses fonctions hydrophiles. Par ailleurs, on peut également incorporer, comme polyholoside, du chitosane, ce qui permet d'obtenir des particules solides ioniquement peu chargées.

Par ailleurs, le couplage chimique covalent selon l'invent permet aussi d'obtenir des greffages fortement liés stables et fixés, dans un sens choisi, par exemple et avantageusement, pour la fixation de peptides, ce qui permet d'obtenir une efficacité de réaction maximale.

D'autres buts, caractéristiques et avantages de l'invention apparaîtront clairement à la lumière de la description explicative qui va suivre, faite en référence à plusieurs exemples de réalisation de l'invention donnés simplement à titre d'illustration, et qui ne saurait donc en aucune façon limiter la portée de l'invention.

Dans les exemples, tous les pourcentages sont donnés en poids sauf indication contraire.

### Exemple 1 selon l'invention

Couplage d'anticorps polyclonaux à des particules solides magnétiques par l'EDC (1-éthyl-3-(3-diméthylaminopropyl)carbodiimide) : application à la détection du rotavirus humain dans les selles.

### 1. Préparation des particules solides (sous forme de microcapsules)

Principe :

Des particules solides de taille moyenne de 50 $\mu$m environ ont été préparées et réticulées après émulsion d'une solution aqueuse d'atélocollagène et de chitosane dans une phase huileuse. Cette préparation a été effectuée selon le procédé décrit dans l'exemple 1 de FR-B1-2 642 329 (89-01221). L'incorporation d'oxyde de fer magnétique (FE304) permet l'obtention d'une phase solide séparable par action d'un champ magnétique.

Mode opératoire :

A une solution d'atélocollagène à 1,6% est ajoutée 10% d'une solution de KITAN® à 3% dans l'acide lactique. L'atélocollagène est préparé selon la méthode décrite dans le brevet français n° 8901221.

Le KITAN® est fourni par la Société ABER TECHNOLOGIES, Prat Menau 29880 Plouguerneau France. Le KITAN® est un chitosane de masse moléculaire 1 200 000 daltons, possédant un taux de désacétylation de 2,4%.

Au mélange atélocollagène - Kitan® est ajoutée une concentration de 5% de carbonate de sodium. Le pH est alors ajusté à 8,0 par HCl N/10. Dans cette préparation, 1% d'oxyde de fer $Fe_3O_4$ est introduit. 100 g du mélange ainsi obtenu sont émulsionnés dans 300 ml de DRAGOXAT® vendu par la Société allemande DRAGOCO® et contenant 5% de SPAN 85® vendu par ICI COMPANY. L'émulsion est obtenue par agitation rapide à l'aide d'un système agitateur Ultra Turax R tournant à 15 000 tr/min (rpm) pendant 2 min.

400 Ml de DRAGOXAT® renfermant l'agent réticulant, soit 2,5% de chlorure d'acide téréphtalique sont ajoutés à l'émulsion maintenue sous agitation de 15 000 tr/min pendant 30 min.

L'agent réticulant est éliminé grâce à une centrifugation à 1 500 g pendant 10 min et par rejet du surnageant obtenu.

Trois lavages successifs sont effectués avec 400 ml de DRAGOXAT®, les capsules collagéniques étant récupérées par centriguation dans les mêmes conditions que précédemment. Six lavages mettant en oeuvre chaque fois 600 ml d'éthanol sont réalisés afin d'éliminer le DRAGOXAT® résiduel. Deux lavages par 60 ml d'une solution de soude à pH 9 et trois lavages par de l'eau désionisée permettent d'éliminer les agrégats de capsules. Un culot de particules solides est ainsi obtenu après centrifugation à 1 500 g pendant 10 min.

Ce culot est alors dispersé dans un volume égal d'eau désionisée renfermant à 0,01% de mercurothiolate de sodium. Cette dispersion de particules solides sera celle mise en oeuvre lors des détections et dosages décrits ci-dessous.

## 2. Préparation d'un réactif spécifique pour un dosage par immuno-essai

On utilise des anticorps antirotavirus du type gamma G qui sont purifiés à partir d'un sérum de lapin hyperimmunisé par une suspension de rotavirus bovin. La présence d'anticorps anti-antigène de groupe du virus permet la détection du virus humain. La pureté des gamma globulines est vérifiée par électrophorèse. Le dosage protéique en poids/volume est effectué selon la technique de Lowry.

Le couplage des anticorps sur les particules solides magnétiques obtenues à l'étape 1 ci-dessus est effectué en utilisant les fonctions carboxyliques (-COOH) du collagène.

Les particules solides sont au préalable lavées dans un tampon de pH voisin de la neutralité (compris entre 7 et 7,5) de faible force ionique et exempt d'amine (par exemple : tampon Hepes 10 mM).

L'activation des particules solides est effectuée par une carbodiimide soluble (par exemple l'EDC (1-éthyl-3-(3-diméthylaminopropyl)carbodiimide) à une concentration comprise entre 5 et 100 mM (par exemple 20 mM) diluée dans le tampon Hepes 10 mM.

La proportion sera de 1 volume de particules solides magnétiques pour 1 volume d'EDC. L'activation sera faite sous agitation modérée (par exemple sur agitateur rotatif) de la suspension pendant 1 h entre 20 et 25°C. L'excès d'EDC est ensuite éliminé par lavage des particules solides en tampon Hepes 10 mM (4 lavages successifs par ajout de 9 volumes de tampon) et récupération des particules solides par centrifugation ou par magnétisme.

Le couplage des gamma G de lapin (au préalable dialysé en solution de Na 0,15 M) est effectué en ajoutant au dernier culot de centrifugation 1 volume de tampon carbonate-bicarbonate 0,1 M et 1 mg de gamma G par ml de culot. Le couplage est effectué à température ambiante sur agitateur rotatif durant une heure.

Le blocage des fonctions résiduelles est effectué par ajout de 10% du volume total d'une solution d'éthanolamine 1 M pH 9,0 et agitation durant 1 h entre 20 et 25°C.

Une série de 4 lavages avec un tampon Tris 50 mM en final permet d'éliminer l'excès d'anticorps n'ayant pas réagi et la remise en suspension à 10% dans ce tampon permet d'obtenir la suspension définitive du réactif spécifique recherché.

## 3. Détection d'un élément spécifique (formant réactif obtenu à l'étape 2 ci-dessus)

Cette préparation a pu être testée vis-à-vis des selles humaines étiquetées positives et négatives pour la recherche du rotavirus. Cette classification préalable a pu être réalisée à l'aide d'un coffret réactif de type ELISA classique disponible sur le marché. Le protocole de la réaction immunologique avec le réactif de particules solides magnétiques ci-dessus est le suivant :
- dilution des selles au 3/30 en tampon Tris 50 mM - pH 7,4. Homogénéisation par agitation puis centrifugation 5 min pour décanter la matière fécale ;
- mise en présence de 400 μl de surnageant et 40 μl de réactif de particules solides magnétiques à 10% ;
- agitation 20 min sur agitateur oscillant ;

- lavages rapides par 1 ml de tampon PBS (Phosphate Buffer Saline) - Tween® et séparation de la phase solide par aimantation ;
- mise en contact du réactif de particules solides récupéré avec 500 µl d'un anticorps monoclonal anti-rotavirus dit "révélateur" car conjugué à la peroxydase ;
- agitation 20 min sur agitateur oscillant ;
- lavages rapides par 1 ml de tampon PBS - Tween®, séparation des phases par aimantation ;
- révélation de l'activité enzymatique par un substrat chromogène : $H_2O_2$/orthophénylènediamine.

La lecture est effectuée visuellement, l'intensité de la coloration appréciée en +, cf. le tableau I ci-dessous.

### Tableau I

| Selle | Coloration | Interprétation |
|-------|------------|----------------|
| A | 0 | négative |
| B | ++++ | positive |
| C | ++++ | positive |
| D | 0 | négative |

### Exemple 2 selon l'invention

Couplage d'anticorps polyclonaux à des particules solides magnétiques par l'EDC : application au titrage de l'HCG (Human Chorionic Gonadotrophin) dans le sérum.

Les anticorps anti HCG utilisés sont du type gamma G et sont purifiés à partir de sérum de lapin hyperimmunisé par l'HCG purifiée totale. Cet antisérum comporte donc des anticorps de spécificité $\alpha$ et $\beta$. Le couplage a été effectué selon le même protocole que dans l'exemple 1,1 et 1,2.

La préparation formant réactif a pu être testée vis-à-vis de sérums humains de femmes enceintes classés au préalable à l'aide d'un coffret ELISA classique du commerce. Deux sérums de donneurs masculins servent de témoin négatif.

Le protocole de la réaction immunologique avec les particules solides magnétiques sensibilisées est le suivant :
- mise en présence de 50 µl de sérum non dilué et 40 µl de réactif de particules solides magnétique à 10% ;
- agitation 15 min sur agitateur oscillant ;
- ajout de 100 µl d'un anticorps monoclonal anti HCG (spécificité $\beta$) conjugué à de la peroxydase ;
- agitation 15 min sur agitateur oscillant
- lavages rapides par 1 ml de tampon PBS - Tween®, séparation des phases par aimantation ;
- révélation de l'activité enzymatique par l'$H_2O_2$/orthophénylènediamine.

La lecture est effectuée sur un spectrophotomètre à 492 nm après blocage de la réaction enzymatique par 1 ml d'acide sulfurique, cf. tableau II ci-dessous :

## Tableau II

### Dosage de l'HCG dans les sérums humains

**II-A**

| Sérums de femmes enceintes | DO (492 nm) |
|---|---|
| 1 | 0,544 |
| 2 | 0,900 |
| 3 | 1,454 |
| 4 | 0,139 |
| 5 | 0,443 |

**II-B**

| Sérums de donneurs masculins | |
|---|---|
| a | 0,096 |
| b | 0,106 |
| Sérums étalon 5 mUI[*]<br>(seuil de positivité) | 0,153 |

[*] mUI = milli unité internationale

### Conclusion

La DO des 5 sérums positifs est proportionnelle à la concentration de l'HCG présente.

Les sérums de donneurs masculins sont bien négatifs (< seuil).

Les particules solides magnétiques sont utilisables dans le cadre d'une détermination quantitative.

### Revendications

1. Utilisation d'atélocollagène ou d'un mélange atélocollagène-polyholoside, comme support solide de fixation d'un capteur, dénommé ligand ou molécule d'affinité, capable de réagir spécifiquement avec un élément à détecter dans un milieu biologique, pour former un complexe spécifique pouvant être détecté par tout moyen de détection.

2. Utilisation selon la revendication 1, caractérisée en ce que l'atélocollagène ou le mélange atélocollagène-polyholoside est sous forme de particules, billes ou micro-capsules, de préférence d'un diamètre compris entre 5 et 500 µm.

3. Utilisation selon la revendication 1 ou 2, caractérisée en ce que l'atélocollagène est combiné à un polyholoside, la proportion relative de polyholoside par rapport à l'atélocollagène étant avantageusement comprise entre 15 et 50 % en poids.

4. Utilisation selon la revendication 2 ou 3, caractérisée en ce que les particules, billes ou microcapsules

EP 0 598 014 B1

précitées contiennent un oxyde magnétique, de préférence un oxyde magnétique de fer.

5. Utilisation selon la revendication 4, caractérisée en ce que la concentration en oxyde magnétique est comprise entre 0,1 et 10 % en poids.

6. Utilisation selon l'une des revendications 1 à 5, caractérisée en ce qu'un capteur est fixé de manière covalente par un agent chimique, au support solide.

7. Utilisation selon la revendication 6, caractérisée en que la fixation est réalisée avec des fonctions aminées et/ou avec des fonctions carboxyliques et/ou avec des fonctions alcooliques de l'atélocollagène et/ou du polyholoside.

8. Utilisation selon l'une des revendications 1 à 7, caractérisée en ce que le polyholoside est soit un glycosaminoglycane, avantageusement de structure et choisi parmi le groupe constitué par le chondroïtine-4-sulfate, le chondroïtine-6-sulfate, le dermatane-sulfate, l'heparane-sulfate, le keratane-sulfate et l'héparine et ses dérivés, en particulier les héparines de bas poids moléculaire ayant un poids moléculaire compris entre 2 000 et 10 000, soit le chitosane, soit encore le dextrane.

9. Utilisation selon l'une des revendications 1 à 8, caractérisée en ce que le capteur est choisi parmi un anticorps, un antigène, sous forme soluble ou particulaire, en particulier un anticorps anti-rotavirus humain ou un anticorps anti-HCG.

10. Utilisation selon l'une des revendications 1 à 9, caractérisée en ce que le support solide est à spécificité multiple.

11. Réactif biologique, caractérisé en ce qu'il comprend un support solide fait d'atélocollagène ou d'un mélange d'atélocollagène et de polyholoside, sur lequel est fixé un capteur formant ligand ou molécule d'affinité, spécifique d'un élément à détecter dans un milieu biologique.

12. Réactif selon la revendication 11, caractérisé en ce que le support solide est tel que défini dans l'une quelconque des revendications 2 à 10.

13. Procédé de détection d'un élément présent dans un milieu biologique, caractérisé en ce qu'on utilise comme réactif biologique un réactif biologique selon la revendication 11 ou 12.

14. Procédé selon la revendication 13 pour la détection d'un rotavirus humain, caractérisé en ce qu'on utilise un réactif biologique dont le capteur comprend un anticorps anti-rotavirus humain.

15. Procédé selon la revendication 13 pour le titrage de l'HCG dans le sérum, caractérisé en ce qu'on utilise un réactif biologique dont le capteur comprend un anticorps anti-HCG.

**Patentansprüche**

1. Verwendung von Atelokollagen oder einer Atelokollagen-Stärkehydrolysat-Mischung als festen Träger zur Bindung eines als Liganden bezeichneten Fängers oder eines Affinitätsmoleküls, der bzw. das spezifisch mit einem in einem biologischen Medium zu detektierenden Element reagieren kann, wobei ein spezifischer Komplex gebildet wird, der durch jedes Detektionsmittel detektiert werden kann.

2. Verwendung nach Anspruch 1, dadurch gekennzeichnet, daß das Atelokollagen oder die Atelokollagen-Stärkehydrolysat-Mischung in Form von Teilchen, Kügelchen oder Mikrokapseln vorzugsweise mit einem Durchmesser zwischen 5 und 500 μm vorliegt.

3. Verwendung nach Anspruch 1 oder 2, dadurch gekennzeichnet, daß das Atelokollagen mit einem Stärkehydrolysat kombiniert wird, wobei die relative Menge an Stärkehydrolysat, bezogen auf das Atelokollagen, vorteilhaft zwischen 15 und 50 Masse-% beträgt.

4. Verwendung nach Anspruch 2 oder 3, dadurch gekennzeichnet, daß die Teilchen, Kügelchen oder Mikro-

9

kapseln ein magnetisches Oxid, vorzugsweise ein magnetisches Eisenoxid, enthalten.

5. Verwendung nach Anspruch 4, dadurch gekennzeichnet, daß die Konzentration an magnetischem Oxid zwischen 0,1 und 10 Masse-% beträgt.

6. Verwendung nach einem der Ansprüche 1 bis 5, dadurch gekennzeichnet, daß ein Fänger durch ein chemisches Mittel an den festen Träger kovalent gebunden wird.

7. Verwendung nach Anspruch 6, dadurch gekennzeichnet, daß die Bindung des Atelokollagens und/oder Stärkehydrolysats mit Amin-Funktionen und/oder mit Carboxyl-Funktionen und/oder mit Alkohol-Funktionen durchgeführt wird.

8. Verwendung nach einem der Ansprüche 1 bis 7, dadurch gekennzeichnet, daß das Stärkehydrolysat entweder ein Glykosaminoglykan, vorteilhaft mit der Struktur von und ausgewählt aus der Gruppe bestehend aus Chondroitin-4-sulfat, Chondroitin-6-sulfat, Dermatansulfat, Heparansulfat, Keratansulfat und Heparin und seinen Derivaten, insbesondere den Heparinen mit geringer Molmasse, mit einer Molmasse zwischen 2 000 und 10 000, oder Chitosan oder auch Dextran ist.

9. Verwendung nach einem der Ansprüche 1 bis 8, dadurch gekennzeichnet, daß der Fänger aus einem Antikörper oder einem Antigen, in löslicher oder Teilchenform, insbesondere einem Human-anti-Rotavirus-Antikörper oder einem anti-HCG-Antikörper, ausgewählt wird.

10. Verwendung nach einem der Ansprüche 1 bis 9, dadurch gekennzeichnet, daß der feste Träger eine mehrfache Spezifität aufweist.

11. Biologisches Reagens, dadurch gekennzeichnet, daß es einen aus Atelokollagen oder einer Mischung von Atelokollagen und Stärkehydrolysat bestehenden festen Träger, an den ein einen Liganden bildender Fänger oder ein spezifisches Affinitätsmolekül eines in einem biologischen Medium zu detektierenden Elements gebunden ist, umfaßt.

12. Reagens nach Anspruch 11, dadurch gekennzeichnet, daß der feste Träger wie in einem der Ansprüche 2 bis 10 definiert ist.

13. Verfahren zur Detektion eines in einem biologischen Medium vorliegenden Elements, dadurch gekennzeichnet, daß als biologisches Reagens ein biologisches Reagens nach Anspruch 11 oder 12 verwendet wird.

14. Verfahren nach Anspruch 13 zur Detektion eines Human-Rotavirus, dadurch gekennzeichnet, daß ein biologisches Reagens, dessen Fänger einen Human-anti-Rotavirus-Antikörper umfaßt, verwendet wird.

15. Verfahren nach Anspruch 13 zur Titrierung von HCG im Serum, dadurch gekennzeichnet, daß ein biologisches Reagens, dessen Fänger einen anti-HCG-Antikörper umfaßt, verwendet wird.

**Claims**

1. Use of atelocollagen or an atelocollagen-polyholoside mixture, as solid binding substrate for a sensor, named as ligand or refining molecule, capable of specifically reacting with an element to be detected in a biological medium to form a specific complex which can be detected by any detection means.

2. Use according to claim 1, characterized in that the atelocollagen or atelocollagen-polyholoside mixture is in the form of particles, spheres or microcapsules, preferably of a diameter ranging between 5 and 500 μm.

3. Use according to claim 1 or 2, characterized in that the atelocollagen is combined to a polyholoside, the relative proportion of polyholoside with respect to the atelocollagen advantageously ranging between 15 and 50% by weight.

4. Use according to claim 2 or 3, characterized in that said particles, spheres or microcapsules contain a magnetic oxide, preferably a magnetic iron oxide.

5. Use according to claim 4, characterized in that the magnetic oxide concentration is comprised between 0.1 and 10% by weight.

6. Use according to one of claims 1 to 5, characterized in that a sensor is covalently bonded by a chemical agent, to the solid substrate.

7. Use according to claim 6, characterized in that the bonding is performed with amino functions and/or with carboxylic functions and/or with alcoholic functions of the atelocollagen and/or of the polyholoside.

8. Use according to one of claims 1 to 7, characterized in that the polyholoside is either an advantageous structural glycosaminoglycan, selected from the group consisting of chondroitin-4-sulfate, chondroitin-6-sulfate, dermatan-sulfate, heptaran-sulfate, ketaran-sulfate and heparin and its derivatives, in particular the heparins of low molecular weight whose molecular weight is comprised between 2,000 and 10,000, or chitosan, or even dextran.

9. Use according to one of claims 1 to 8, characterized in that the sensor is selected from an antibody, an antigen, in soluble or particulate form, in particular a human anti-rotavirus antibody or an anti-HCG antibody.

10. Use according to one of claims 1 to 9, characterized in that the solid substrate has multiple specificity.

11. Biological reactant, characterized in that it comprises a solid substrate made of atelocollagen or of a mixture of atelocollagen and of polyholoside, on which is bonded a sensor forming ligand or refining molecule, specific of an element to be detected in a biological medium.

12. Reactant according to claim 11, characterized in that the solid substrate is as defined in any one of claims 2 to 10.

13. Method for the detection of an element present in a biological medium, characterized in that the biological reactant used is a biological reactant according to claim 11 or 12.

14. Method according to claim 13 for detecting a human rotavirus, characterized in that it uses a biological reactant whose sensor comprises a human anti-rotavirus antibody.

15. Method according to claim 13 for assaying HCG in the serum, characterized in that it uses a biological reactant whose sensor is an anti-HCG antibody.